# EUROPÄISCHE PATENTSCHRIFT

(11) **EP 4 085 833 B1**
(45) Veröffentlichungstag und Bekanntmachung des Hinweises auf die Patenterteilung: **05.03.2025**
(21) Anmeldenummer: 22171623.6
(22) Anmeldetag: 04.05.2022
(51) Int. Cl.: A61B 5/097, A61B 5/08, G01N 33/497

(54) **BEHÄLTER, NACHWEISGERÄT SOWIE EIN VERFAHREN FÜR IN LUFT ENTHALTENE PATHOGENE SUBSTANZEN**
CONTAINER, DETECTION DEVICE AND METHOD FOR PATHOGENIC SUBSTANCES IN AIR
RÉCIPIENT, APPAREIL DE DÉTECTION, AINSI QUE PROCÉDÉ POUR SUBSTANCES PATHOGÈNE CONTENUES DANS L'AIR

(30) Priorität: 04.05.2021 DE 102021111494
(43) Veröffentlichungstag der Anmeldung: 09.11.2022
(73) Patentinhaber: HAGER SAFETY Deutschland GmbH, 66440 Blieskastel (DE)
(72) Erfinder: Schaaf, Norbert, 65343 Eltville (DE); Kroh, Christoph, 65510 Idstein (DE); Viertel, Tobias, 65527 Niederseelbach (DE)
(74) Vertreter: WSL Patentanwälte Partnerschaft mbB

(56) Entgegenhaltungen:
- WO-A1-2018/004603
- WO-A1-2020/160753
- US-A1- 2008 166 792
- US-A1- 2010 279 271
- US-A1- 2013 303 929
- US-A1- 2016 199 603
- US-B2- 9 522 250

## Beschreibung

Die Erfindung betrifft ein Nachweisgerät sowie ein Nachweisverfahren für den vorzugsweise quantitativen Nachweis in Luft enthaltener pathogener Substanzen, insbesondere Viren. Die Erfindung betrifft ferner einen Behälter mit einem Lufteinlass, einem Luftauslass und einem Nachweisvolumen zur Aufnahme eines Nachweismediums.

Der in situ-Nachweis pathogener Substanzen in der Raum- und Atemluft, sogenannter Aerosolpartikel, ist seit langem Gegenstand der Forschung hat nicht zuletzt durch die pandemischen Folgen des zuletzt in Erscheinung getretenen SARS-CoV-2 Virus noch einmal besonders an Fahrt aufgenommen. In Folge dessen gibt es zahlreichen auch jüngeren Stand der Technik. Beispielhaft wird auf folgende Schriften verwiesen.

Das Dokument CN 11 1662816 A offenbart eine Anordnung, die Raumluft ansaugt, in einem Anreicherungsbehälter Viren herausfiltert und diese zusammen mit einer Pufferlösung zu einem mikrofluidischen Chip leitet, in dem unter Temperaturkontrolle eine biochemische Reaktion (LAMP) stattfindet und mittels optischer Verfahren nachgewiesen wird.

Aus der US 2020/0309703 A1 ist ein Aerosoldetektor bekannt, der auf einer Fluoreszenzanregung von schädlichen Aerosolpartikeln, insbesondere auch SARS-CoV-2 Viren, mittels UV-Strahlung basiert. Die Partikel werden durch elektrostatische Kraft oder einfach durch die Gravitationskraft auf einer transparenten Platte aufgefangen und gehalten. Die Platte ist mittels UV-LED hinterleuchtet. Fluoreszenz wird durch Betrachten der Platte wahrgenommen oder kann mittels einem nicht näher beschriebenen Detektor erfasst werden. Bei Erfassen von Fluoreszenzstrahlung kann ein Alarm ausgelöst werden.

Aus der WO 2008/108872 A2 ist ein Detektor zum Nachweisen unterschiedlicher bioaktiver Substanzen, unter anderem von Influenza Typ A-Viren bekannt. Der Nachweis bestimmter Antigene erfolgt mittels Antikörperreaktion. Ein Sensorelement ist zu diesem Zweck mit dem Antikörper Ligand beschichtet. Gemessen wird wahlweise ein für die Reaktion charakteristischer elektrostatischer Puls mittels einer Elektrode (bio pore sensor) oder eine charakteristische Fluoreszenz mittels Photodetektor (optical based sensor).

Die US 7,265,669 B2 behandelt eine selbstregenerierende Kollektorplatte für nachzuweisende Partikel. Als Nachweispartikel sind unter anderem auch Viren genannt. Zum Detektionsverfahren wird erwähnt, dass die Vorrichtung eine Anregungslichtquelle und einen Photodetektor für Fluoreszenzlicht umfasst. Der Detektor kann zwecks Raumluftüberwachung an der Wand oder Decke montiert werden.

In der Schrift US 7,494,769 B2 werden abermals Bioaerosole mittels Fluoreszenz- oder Phosphoreszenzmessung detektiert. Die Raumluft wird zu einem Kollektor gepumpt. Eine Besonderheit ist ein Kollektor mit Flüssigkeitsreservoir, in dem die Aerosole gefangen und konzentriert werden.

In dem Dokument CN 11 1665356 A geht es spezifisch um den Nachweis von Covid-19-Viren. Das labortechnische Analyseverfahren basiert auf der Raman-Spektroskopie, genauer surface enhanced Raman Spectroscopy (SERS). Analysiert werden Fluidproben, die beispielsweise aus einem konzentrierten Aerosol bestehen können. Für die SERS werden Silber- oder Goldoberflächen verwendet, die mit Antikörpern funktionalisiert werden.

Die WO 2006/106235 A1 hat eine Vorrichtung und ein automatisiertes Nachweisverfahren für mikrobiologisches Material, unter anderem Viren, zum Gegenstand, bei dem ein Aerosol angesaugt zu einer Probe aufkonzentriert und einer Anordnung von Testscheiben zugeführt wird, die in Testzonen mit Antikörpern beschichtet sind. Eine Reaktion verursacht eine Verfärbung der Testzone, welche automatisch optisch ausgelesen und einem Auswertesystem zugeführt wird. Abgesehen von einer automatischen Auslesung erinnert die Nachweismethode an die aus den Schnelltests bekannte Immunfärbung.

Dem Dokument CN 11 0118711 B zufolge, wird die bakterielle Belastung der Raumluft anhand von Fluoreszenzmessungen unter Berücksichtigung verschiedener Umgebungsbedingungen ermittelt. Die darin beschriebene Vorrichtung ist geeignet infolge der Detektion ein Warnsignal auszugeben.

In der CN 20 7516253 U ist eine Vorrichtung zum Nachweis von Hepatitis-B-Viren in der Luft offenbart. Die Vorrichtung saugt Luft in ein Gehäuse ein und leitet sie in eine dort befindliche Testflüssigkeit. Die Flüssigkeit wird über ein Kanalsystem auf mehrere Teststreifen aufgebracht und die Teststreifen können durch Fenster in der Gehäusewand beobachtet werden.

Auch die Schrift CN 21 0720420 U befasst sich mit dem Nachweis pathogener Substanzen in der Luft mittels Antikörpern, die auf einem Harz aufgetragen sind, und mit dem Stopfbuchsen gefüllt sind.

Eine andere Vorrichtung zum Erkennen bioaktiver Substanzen in der Luft ist aus der Schrift EP 1 309 720 B1 bekannt. Die Vorrichtung umfasst einen Einlass, einen Filter zum Herausfiltern von Staub und größeren Partikeln, eine Fluidleitung mit einer Barriere, an der sich ein Wirbel zum Sortieren von Partikeln nach Größe bildet, und eine Biorezeptorfläche, auf der die Partikel auftreffen und die mit einem Aptamer zum Nachweis bestimmter Substanzen durch Bindung versehen ist. Auch hier wird die Reaktion anhand von Fluoreszenzstrahlung optisch erfasst.

Wie in der Schrift zuvor hat auch die US 2019/0242807 A1 eine Vorrichtung und ein Verfahren zum schnellen in situ-Nachweis von Pathogenen, insbesondere Viren, in der Raumluft mittels Aptamer zum Gegenstand. Die Vorrichtung saugt Luft an, reinigt diese von Partikeln und reichert sie in einer Pufferlösung an. Diese wird in eine Detektionskammer gepumpt, die eine mit einem Aptamer funktionalisierte Elektrode aufweist. Parallel zu einer Vielzahl von Detektionskammern sind Gegenelektroden angeordnet. Der Nachweis von Pathogenen erfolgt anhand einer charakteristischen elektronischen Antwort auf ein dazwischen angelegtes elektrisches Feld.

In der EP 1 882 177 B1 ist die Reaktion eines luftgetragenen Partikels mit einem "Reporter" beschrieben, die nach erfolgter Reaktion in einer Detektionszone mit Licht bestrahlt werden. Als Reaktion werden Fluoreszenz, Phosphoreszenz etc. genannt. Der Reporter ist ein fluoreszierendes oder phosphoreszierendes Molekül, welches an ein Antigen gebunden sein kann.

In der Schrift US 10,677,773 B2 geht es allgemein um eine miniaturisierte Vorrichtung zur Überwachung der Umgebungsluft. Ein Virusdetektor ist beispielhaft genannt. Es geht dabei inhaltlich im Wesentlichen um die räumliche Anordnung von Pumpen, Kanälen, Sensoren, Stromversorgung etc. in der Vorrichtung.

Die US 7,705,739 B2 offenbart ein Analyse- und Warnsystem für eine mehrstufige Detektion verschiedener pathogener Stoffe, u. a. Viren, in der Luft. Das System umfasst ein optisches Detektionsmodul, welches für eine Lumineszenzmessung bestimmt ist.

Die Schrift EP 1 158 292 B1 befasst sich mit einem Aerosolpartikeldetektor basierend auf einer winkelaufgelösten Streulichtmessung unter Inzidenz eines Laserstrahls.

Ähnlich befasst sich auch die US 7,053,783 B2 mit einem Streulichtdetektor, speziell zur Ermittlung pathogener Partikel in der Luft. Ermittelt wird allein die Pulshöhe der Streulichtsignale, die jeweils einer Partikelgröße zugeordnet wird, wobei aus dem Auftreten bestimmter Größen auf das Vorhandensein pathogener Substanzen geschlossen wird.

Aus der KR 101625133 B1 ist ein Luftqualitätsmesssystem mit vernetzten Detektoren bekannt, das geeignet sein soll, verschiedene Arten von Luftverunreinigungen festzustellen und zu alarmieren, falls bestimmte Grenzwerte überschritten sind. Unter anderem sind auch Viren als Verunreinigungen genannt. Der Schwerpunkt der Schrift liegt in der Vernetzung. Über die Detektion ist aus den übersetzten Ausschnitten nichts ausgesagt.

Das Dokument US 2013/303929 A1 offenbart einen Behälter für den quantitativen Nachweis in Luft enthaltener pathogener Substanzen, insbesondere Viren nach dem Oberbegriff des Anspruchs 1.

Aufgabe der vorliegenden Erfindung ist es, ein verlässliches Nachweisgerät für den in situ-Nachweis in Luft enthaltener pathogener Substanzen bereitzustellen, das aus einfachen Komponenten aufgebaut ist, und verschiedene Anwendungsmöglichkeiten eröffnet.

Die Aufgabe wird gemäß einem ersten Aspekt der Erfindung gelöst durch einen Behälter nach Anspruch 1. Die vorliegende Erfindung bezieht sich ebenfalls auf ein Nachweisgerät nach Anspruch 3, das den erfindungsgemäßen Behälter umfasst.

Der Behälter ist zur Verwendung in einem vorstehend beschriebenen Nachweisgerät eingerichtet und bestimmt. Er weist einen Lufteinlass, einen Luftauslass, ein Nachweisvolumen und ein Nachweismedium für den Nachweis in Luft enthaltener pathogener Substanzen, insbesondere Viren, auf, wobei das Nachweismedium so in dem Nachweisvolumen platziert ist, dass eine Luftströmung innerhalb des Behälters vom Lufteinlass zum Luftauslass das Nachweismedium durchströmt.

Die Aufgabe wird gemäß einem zweiten Aspekt der Erfindung gelöst durch ein Verfahren nach Anspruch 15.

Das Verfahren zum Nachweis in Luft enthaltener pathogener Substanzen, insbesondere Viren, umfasst die Schritte:
- Bereitstellen eines Behälters mit einem Lufteinlass, einem Luftauslass und einem Nachweisvolumen,
- Bereitstellen eines Nachweismediums in dem Nachweisvolumen,
- Erzeugen eines Unterdruckes (bezogen auf den Umgebungsdruck) in einem Ansaugvolumen stromabwärts des Nachweisvolumens mittels einer Pumpe,
- Hindurchsaugen von Luft vom Lufteinlass durch das Nachweismedium hindurch,
- und Detektieren einer Trübung und/oder Färbung in dem Nachweismedium mittels eines Sensorelements in Abhängigkeit von der Präsenz wenigstens einer pathogenen Substanz in der hindurchgesaugten Luft.

"Unterdruck" bezeichnet hierin den Differenzdruck zwischen dem Umgebungsdruck, das heißt der Druck in der umgebenden Raumluft, und dem spezifischen mittels Pumpe erzeugten Druck, wobei letzterer Druck stets geringer als der Umgebungsdruck ist. Die "Pumpe zum Erzeugen eines Unterdruckes" ist dementsprechend eine als Saugpumpe konfigurierte Pumpe, deren Saugseite an das Ansaugvolumen angeschlossen ist und deren Druckseite mittelbar oder unmittelbar mit der Umgebung kommuniziert, um die Luft aus dem Ansaugvolumen anzusaugen und in die Umgebung abzugeben.

Ein wesentlicher Vorteil der Erfindung besteht darin, dass die zu untersuchende Luft durch das Nachweismedium hindurch gesaugt wird, wodurch die pathogenen Substanzen in dem Nachweismedium nicht nur nachgewiesen, sondern auch zugleich aus der Luft herausgefiltert werden. Das Nachweisvolumen steht dabei ziemlich zu Beginn des Strömungsverlaufs, so dass die meisten passiven und aktiven Komponenten der Vorrichtung, insbesondere jene zur Erzeugung und Regulierung der Luftströmung (Pumpe, Ventile, etc.), nicht mit den pathogenen Substanzen in Kontakt kommen und daher seltener bzw. nicht regelmäßig dekontaminiert oder ausgetauscht werden müssen. Hierdurch lässt sich die Vorrichtung einfach und kostengünstig herstellen und betreiben.

Erfindungsgemäß ist das Nachweisvolumen zur Aufnahme einer Nachweisflüssigkeit eingerichtet, wobei der Lufteinlass des Behälters bei bestimmungsgemäßem Gebrauch, bezogen auf die Schwerkraftrichtung, unterhalb des Luftauslasses in den Behälter mündet.

Auf diese Weise wird sichergestellt, dass eine bis zu einem bestimmten Füllstand in den Behälter gefüllte Nachweisflüssigkeit die Lufteinlassmündung fluidisch von der Luftauslassmündung trennt. Der Füllstand definiert demgemäß das Nachweisvolumen in dem Behälter. Der Lufteinlass mündet dann in das Nachweisvolumen, während der Luftauslass oberhalb des Füllstands und damit oberhalb des Nachweisvolumens in den Behälter mündet bzw. von diesem abgeht.

Angaben wie "oberhalb", "über", "unterhalb" oder "unter" beziehen sich hierin einerseits stets auf eine bestimmungsgemäß ausgerichtete Anordnung der Vorrichtung bzw. der darin enthaltenen Bauteile und Substanzen und andererseits auf die Schwerkraftrichtung, wenn nichts anderes angegeben sein sollte.

Als Nachweismedien kommen bevorzugt Flüssigkeiten mit darin enthaltenen Antikörpern, also Antikörperlösungen, in Betracht. Als Antikörper werden hierin alle immunologisch aktiven Proteine, unabhängig von Ihrer Produktion, verstanden, also insbesondere auch monoklonale Antikörper. Eine Variante hiervon sieht eine Nachweisflüssigkeit vor, die mit Antikörpern beladene Latexpartikel enthält. Diese Flüssigkeit wird bekanntermaßen für partikelverstärkte nephelometrische Immunoassays verwendet. Werden die beladenen Latexpartikel mit dem zu messenden Analyten, vorliegend also den Aerosolpartikeln, in Kontakt gebracht, entstehen Agglutinate, die im Falle der Nephelometrie mittels Streulichtmessung quantitativ erfasst werden. Je höher die Konzentration des Analyten, desto zahlreicher und größer sind die streuenden Partikelagglutinate und umso höher ist das Streulichtsignal. Das Prinzip ist bereits seit langem bekannt und beispielsweise in der EP 0 019 741 B1 beschrieben.

Der Lufteinlass wird vorteilhafter Weise durch eine bei bestimmungsgemäßem Gebrauch abschnittsweise, bezogen auf die Schwerkraftrichtung, nach unten in das Nachweisvolumen eintauchende Zuleitung (Tauchrohr) gebildet.

Mit dieser Anordnung wird nach dem Prinzip einer Gaswaschflasche die Luft von oben zugeführt und unmittelbar in der Nachweisflüssigkeit freigesetzt, von wo diese durch die Nachweisflüssigkeit perlt und die enthaltenen Aerosole mit der Nachweisflüssigkeit in Kontakt treten. Bevorzugt befindet sich im Bereich des Lufteinlasses in dem Behälter eine poröse oder perforierte Luftverteilerstruktur.

Eine solche Struktur dient der Verteilung der in das Nachweisvolumen strömenden Luft auf möglichst kleine Gasblasen, um die gesamte Kontaktfläche zwischen der Luft und der Nachweisflüssigkeit zu vergrößern. Als sekundärer Effekt verringert sich die Aufstiegsgeschwindigkeit der Blasen, wenn diese kleiner sind, wodurch sich zugleich die Kontaktzeit zwischen der Luft und der Nachweisflüssigkeit verlängert. Ziel ist es grundsätzlich die Reaktion zwischen der pathogenen Substanz und der Nachweisflüssigkeit zu optimieren, so dass beispielsweise möglichst jeder Virus mit mindestens einem Antikörper reagiert.

Die poröse Luftverteilerstruktur wird bevorzugt durch eine granulare Substanz oder ein offenporiges Material gebildet. Zu den granularen Substanzen werden beispielsweise auch Zirkonoxidkugeln gerechnet. Zirkonoxid (ZrO₂) ist zur Herstellung beispielsweise von Hochleistungskeramiken für medizinische Anwendungen bekannt. Durch die Strömung entlang der glatten keramischen Oberfläche der Kugeln erfolgt eine Oberflächenvergrößerung der nunmehr sehr kleinen Luftblasen in der Lösung, deren Anzahl aber erhöht ist. Folglich ist die Gesamtoberfläche der Luftblasen, an der eine Reaktion zwischen den nachzuweisenden pathogenen Substanzen, z. B. Viren, und der Nachweisflüssigkeit, z. B. den Antikörpern in der Lösung, erfolgen kann, erhöht. Vorzugsweise sind die Menge an zugeführtem Aerosol, die Größe, Anzahl und Oberflächenbeschaffenheit der Zirkonoxidkugeln, das Volumen und die Geometrie des Nachweisraumes sowie die Nachweisflüssigkeit derart aufeinander abgestimmt, dass in der Nachweisflüssigkeit Luftblasen mit einem Durchmesser von maximal einem Mikrometer erzeugt werden.

Als offenporiges Material kommen Festkörperschäume oder schwammartige Körper mit vergrößerter Oberfläche in Betracht. Als perforierte Strukturen können beispielweise Sieb- oder Düsenplatten aus Glas oder einem anderen geeigneten Material verwendet werden.

Eine vorteilhafte Ausgestaltung der Erfindung sieht vor, dass das Ansaugvolumen durch einen Unterdrucktank gebildet wird, der über eine Anschlussleitung mit dem Luftauslass des Behälters verbunden ist.

Alternativ ist das Ansaugvolumen in dem Behälter integriert. In diesem Fall bilden das Nachweisvolumen und das Ansaugvolumen bevorzugt gemeinsam das Behältervolumen, wobei das Ansaugvolumen bei bestimmungsgemäßem Gebrauch, bezogen auf die Schwerkraftrichtung, oberhalb des Nachweisvolumens, d. h. im Falle einer Nachweisflüssigkeit über deren Füllspiegel, in dem Behältervolumen gebildet wird und wobei der Luftauslass in das Ansaugvolumen mündet bzw. von diesem abgeht.

Bevorzugt weist das Nachweisgerät eine Steuerung auf, an die die Pumpe elektronisch angeschlossen ist.

Ferner ist in dem Ansaugvolumen vorteilhafterweise ein Drucksensor angeordnet oder mit dem Ansaugvolumen ein Drucksensor unmittelbar fluidisch verbunden, der mit der Steuerung elektronisch verbunden ist. Als "unmittelbar fluidisch verbunden" ist, in Abgrenzung zu einer Verbindung durch das Nachweismedium hindurch, eine Verbindung durch eine offene Fluidleitung gemeint.

Als vorteilhaft hat sich ferner herausgestellt, dass ein steuerbares Pumpenventil vorgesehen ist, das zwischen der Pumpe und dem Ansaugvolumen angeordnet und mit der Steuerung elektronisch verbunden ist. Ein solches Ventil wird benötigt, wenn das Ansaugvolumen beispielsweise aus Platzgründen nicht sehr groß ist und die Pumpe das Ansaugvolumen während eines Messvorgangs daher intermittierend auspumpt. Dann stellt dieses Ventil sicher, dass während des Stillstands der Pumpe keine Umgebungsluft durch die Pumpe zurück in das Ansaugvolumen strömt. Alternativ kann in die Pumpe ein Rückschlagventil integriert sein, welches die gleiche Wirkung hat.

Weiterhin ist vorzugsweise ein Gehäuse vorgesehen, in dem der Behälter angeordnet ist und das einen Einlassstutzen oder eine Einlassöffnung aufweist, der oder die fluidisch mit dem Lufteinlass kommuniziert.

Vorteilhafterweise ist auch ein steuerbares Einlassventil vorgesehen, das stromaufwärts des Lufteinlasses in dem Einlassstutzen angeordnet ist und das mit der Steuerung elektronisch verbunden ist.

Wird das Nachweisgerät beispielsweise zur Analyse des Atems einer Person verwendet, dann öffnet das Ventil qua Steuerung nur, wenn stromaufwärts ein leichter Überdruck detektiert wird, wie nachstehend erläutert wird. Es hat dadurch einen doppelten Nutzen. Zum einen wird Luft nur angesaugt wenn auch ein Proband in ein mit dem Einlassstutzen verbundenes Mundstück atmet. Eine Rückatmung wird somit verhindert und die Voraussetzung für einen definierten Volumenstrom durch die Nachweisflüssigkeit geschaffen. Zum anderen verhindert es ein versehentliches Austreten der Nachweisflüssigkeit, wenn das Nachweisgerät nicht in Betrieb ist, d. h. insbesondere in dem Ansaugvolumen kein Unterdruck ansteht, und wenn das Nachweisgerät nicht in bestimmungsgemäßer Orientierung gehalten oder abgelegt wird.

Weiterhin weist das Nachweisgerät vorteilhafter Weise einen Drucksensor auf, der stromaufwärts des Einlassventils in dem Einlassstutzen angeordnet oder mit diesem unmittelbar fluidisch verbunden ist und der mit der Steuerung elektronisch verbunden ist.

Wird das Nachweisgerät beispielsweise zur Analyse des Atems einer Person verwendet, dient die Kombination aus dem Einlassventil und einem stromaufwärts angeordneten Drucksensor dazu, zunächst festzustellen, dass eine Person in ein mit dem Einlassstutzen verbundenes Mundstück atmet. Hierdurch erhöht sich der vor dem Einlassventil anstehende Druck (Eingangsdruck). Stellt der Sensor fest, dass ein voreingestellter Wert überschritten wird, also ein definierter Überdruck erreicht ist, öffnet die Steuerung das Einlassventil, um das Mundstück fluidisch mit dem Nachweisvolumen zu verbinden. Der definierte Druckabfall zwischen dem so geregelten Überdruck im Mundstück und dem Ansaugvolumen sorgt für einen geregelten Volumenstrom des ausgeatmeten Aerosols und schafft somit die Voraussetzung für eine verlässliche, quantitative Bestimmung der in einem bestimmten Volumen enthaltenen Aerosolpartikel. Unterschreitet der mittels des Drucksensors ermittelte Eingangsdruck den gleichen oder einen anderen voreingestellten Wert, dann schließt die Steuerung das Einlassventil, um das Mundstück von dem Nachweisvolumen zu trennen, und unter anderem eine Rückatmung zu verhindern.

Dementsprechend umfasst das Nachweisgerät in dieser Ausgestaltung ferner vorzugsweise ein mit dem Einlassstutzen verbindbares Mundstück.

Das Mundstück umfasst dabei besonders bevorzugt einen Einlassabschnitt, einen Auslassabschnitt und einen abzweigenden Verbindungsabschnitt, wobei der Verbindungsabschnitt zur fluidischen Verbindung mit dem Einlassstutzen eingerichtet ist. Einlassabschnitt, Auslassabschnitt und Verbindungsabschnitt sind dabei strömungstechnisch so gestaltet und dimensioniert, dass sich einerseits durch Ausatmen in den Einlassabschnitt hinein ein ausreichender Überdruck in dem Verbindungsabschnitt aufbauen lässt, um den steuerungstechnisch voreingestellten Wert zu überschreiten und somit das Einlassventil zu öffnen. Andererseits muss gewährleistet sein, dass sich durch das Ausatmen auch kein wesentlich höherer Überdruck aufbaut, um den Volumenstrom durch das Nachweismedium nicht erheblich zu manipulieren.

Zusätzlich oder alternativ zu dem Drucksensor stromaufwärts des Einlassventils kann ein beispielsweise durch den Probanden zu betätigender Schalter an dem Nachweisgerät vorgesehen sein, der das Gerät in Bereitschaft versetzt und/oder die Messung startet.

Der Auslassabschnitt umfasst dabei vorzugsweise ein die pathogene Substanz zurückhaltendes Filterelement, damit auch die ausgeatmete und das Nachweismedium nicht passierende Luft möglichst frei von etwaig vorhandenen pathogenen Substanzen in die Umgebung abgegeben wird.

Vorzugsweise weisen der Behälter und das Gehäuse korrespondierende Verriegelungselemente auf, wobei der Behälter aus dem Gehäuse entnehmbar ist, wenn die Verriegelungselemente nicht in Eingriff stehen, und in dem Gehäuse in seiner bestimmungsgemäßen Position arretiert ist, wenn die Verriegelungselemente in Eingriff stehen.

Das Nachweisgerät erlaubt somit einen Austausch des Behälters, beispielsweise, wenn dieser eine bestimmte Anzahl von Testzyklen durchlaufen hat oder wenn der Nachweis einer pathogenen Substanz (positiver Befund) erfolgt ist. Dafür umfasst die Steuerung vorteilhafter Weise einen Zähler, der bei jedem erfolgten Testzyklus um Eins inkrementiert wird.

Ein Vorteil ist es ebenfalls, wenn der Behälter am Lufteinlass und am Luftauslass jeweils ein das Austreten des Nachweismediums aus dem entnommenen Behälter verhinderndes Verschlusselement aufweist.

Das Verschlusselement kann als passives Ventil oder Rückschlagventil ausgestaltet sein, dass aufgrund einer Rückstellkraft ohne äußere Krafteinwirkung den Behälter verschlossen hält, wobei es sich im bestimmungsgemäß eingesetzten Zustand und/oder im Betrieb durch Wechselwirkung mit einem Gehäuseelement (auslassseitig) oder durch ein Druckgefälle von außen nach innen (einlassseitig) öffnet.

Bevorzugt umfasst das Gehäuse, soweit vorhanden, die Pumpe, die Steuerung, die steuerbaren Ventile, das Sensorelement und den Einlassstutzen. Das Gehäuse beherbergt somit alle festen, das heißt nicht austauschbaren, Bauteile und Funktionselemente des Nachweisgeräts, während der Behälter als Austauschteil nach jedem positiven Befund oder spätestens nach einer bestimmten Anzahl von Testzyklen austauschbar ist. Das Mundstück wiederum ist als einmal verwendbares Teil konzipiert.

Das Sensorelement ist vorzugsweise ein mit der Steuerung elektronisch verbundener optischen Sensor, wobei in einer Wand des Behälters im Bereich des Nachweisvolumens ein Sichtfenster angeordnet ist, auf das der optische Sensor ausgerichtet ist.

Ferner weist das Nachweisgerät in dem Gehäuse vorzugsweise eine mit der Steuerung elektronisch verbundene Lichtquelle und ein in einer Wand des Behälters im Bereich des Nachweisvolumens angeordnetes Einlassfenster auf, auf das die Lichtquelle ausgerichtet ist.

Dabei sind die Lichtquelle, der optische Sensor und die Steuerung besonders bevorzugt zur Bestimmung des in dem Nachweismedium absorbierten oder gestreuten Lichtes eingerichtet.

Weiterhin hat es sich als vorteilhaft erwiesen, wenn in dem Behälter innerhalb des Nachweisvolumens wenigstens ein Luftleitelement der Gestalt angeordnet ist, dass der Luftstrom vom Lufteinlass zum Luftauslass wenigstens einmal umgelenkt wird, so dass das Nachweismedium nicht auf direktem Weg vom Lufteinlass zum Luftauslass durchströmt wird. Das wenigstens eine Luftleitelement bildet dabei besonders bevorzugt eine mäanderförmige Struktur.

Als Nachweisverfahren kommt die oben schon angesprochene Nephelometrie in Betracht, bei der sich die quantitative Konzentration feinverteilter, kolloidaler Aerosolteilchen über die Trübung bestimmen lässt. Hierbei wird das Nachweismedium in einen Lichtstrahl gebracht, wobei aufgrund der Trübung ein Teil des Lichtes seitlich zum eintretenden Strahl gestreut wird. Das Streulicht tritt durch das Sichtfenster aus dem Nachweisvolumen des Behälters aus und wird gegebenenfalls über optische Komponenten (Linsen, Prismen, Spiegel) auf einen Photodetektor gelenkt. Alle optischen Komponenten und der Detektor werden als Bestandteile des Sensors verstanden. Die Messung des Photodetektors ist direkt proportional der Lichtintensität.

Auch in Betracht kommt das Nachweisverfahren der Turbidimetrie. Hierbei wird die streuungsbedingte Verringerung der Intensität (Extinktion) des durch das Nachweismedium hindurchgehenden Lichtstrahls, also die Transmission, gemessen, wobei die Konzentration der Reaktionsprodukte direkt proportional zur Trübung der Lösung ist.

Die Aufgabe wird gemäß einem vierten Aspekt der Erfindung gelöst durch die Verwendung des Nachweisgerätes der vorstehend beschriebenen Art als Testvorrichtung zum Nachweis in der ausgeatmeten Luft eines Probanden enthaltener pathogener Substanzen, insbesondere Viren, oder als Überwachungsvorrichtung zum Nachweis in der Raumluft enthaltener pathogener Substanzen, insbesondere Viren. Die Testvorrichtung und die Überwachungsvorrichtung können jeweils zusätzliche spezifische funktionale Komponenten umfassen, wie im Fall der Testvorrichtung das Mundstück oder im Fall der Überwachungsvorrichtung einen Ansaugtrichter oder jeweils angepasste Anzeige- oder Datenübertragungsmodule. Insbesondere kann die Überwachungsvorrichtung eine Schnittstelle und/oder ein Funkmodul für einen kabelgebundenen oder funkbasierten Daten- oder Signalaustausch mit einem Server oder einem stationären oder mobilen Endgerät oder einer gleichartigen Überwachungsvorrichtung aufweisen. Diese Ausstattung ist auch für die Testvorrichtung denkbar, die allerdings in der einfachsten Ausgestaltung als mobiles Testgerät auch mit einer eigenen optischen und/oder akustischen Anzeige auskommt. Andererseits kann auch die Überwachungsvorrichtung eine zusätzliche optische und/oder akustische Anzeige aufweisen, um Personen in dem überwachten Raum unmittelbar zu alarmieren.

Die Erfindung wird nachfolgend anhand von Zeichnungen näher erläutert. Es zeigen:
- Figur 1: eine schematische Darstellung eines ersten Ausführungsbeispiels des erfindungsgemäßen Nachweisgeräts als Testvorrichtung für die ausgeatmete Luft eines Probanden;
- Figur 2: eine schematische Darstellung eines zweiten Ausführungsbeispiels des erfindungsgemäßen Nachweisgeräts als Testvorrichtung für die ausgeatmete Luft eines Probanden;
- Figur 3: eine schematische Darstellung eines dritten Ausführungsbeispiels des erfindungsgemäßen Nachweisgeräts als Testvorrichtung für die ausgeatmete Luft eines Probanden und
- Figur 4: eine schematische Darstellung eines vierten Ausführungsbeispiels des erfindungsgemäßen Nachweisgeräts als Überwachungsvorrichtung für Raumluft.

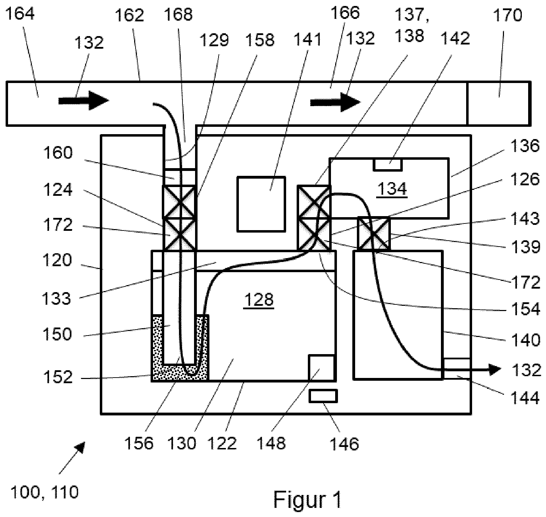

In Figur 1 ist ein Nachweisgerät 100 für den Nachweis in Luft enthaltener pathogener Aerosolpartikel in der Verwendung als Testvorrichtung 110 für die ausgeatmete Luft eines Probanden vereinfacht dargestellt. Es umfasst ein Gehäuse 120, in dem ein Behälter 122 mit einem Lufteinlass 124, einem Luftauslass 126 und einem Nachweisvolumen 128 angeordnet ist. Das Gehäuse 120 weist eine Einlassöffnung 129 auf, die fluidisch mit dem Lufteinlass 124 des Behälters 122 kommuniziert. In dem Nachweisvolumen 128 ist ein Nachweismedium 130, vorzugsweise eine Nachweisflüssigkeit, so aufgenommen, dass eine Luftströmung, siehe Pfeile 132, innerhalb des Behälters 122 vom Lufteinlass 124 zum Luftauslass 126 das Nachweismedium 130 zwangsweise durchströmt. Ist das Nachweismedium 130 eine Nachweisflüssigkeit, befindet sich über deren Füllspiegel 131 vorzugsweise ein luftgefülltes Puffervolumen 133.

In dem Gehäuse 120 ist ferner ein Ansaugvolumen 134 stromabwärts des Nachweisvolumens 128 und innerhalb eines, bezogen auf den Behälter 122, externen Unterdrucktanks 136 angeordnet. Der Unterdrucktank 136 ist genauer über eine Anschlussleitung 137 mit dem Luftauslass 126 des Behälters 122 verbunden. In die Anschlussleitung 137 ist ein steuerbares Ansaugventil 138 eingeschaltet. In dem Gehäuse 120 ist ferner eine mit dem Unterdrucktank 136 über ein steuerbares Pumpenventil 139 verbundene Pumpe 140 zum Erzeugen eines Unterdruckes in dem Ansaugvolumen 134 angeordnet. Die Pumpe 140, das Ansaugventil 138 und das Pumpenventil 139 sind elektronisch an eine Steuerung 141 angeschlossen. Ferner kann ein Drucksensor 142 in dem Ansaugvolumen 134 angeordnet oder mit diesem fluidisch verbunden sein. Der hiermit ermittelte Druck in dem Ansaugvolumen 134 wird mittels der Steuerung 141 mit programmierten Schaltschwellen verglichen, aus welchem Vergleich in Verbindung mit etwaigen weiteren Eingangsgrößen die Steuerung 141 Ein- und Ausschaltsignale für die Pumpe 140 und Steuersignale zum Öffnen oder Schließen des Ansaugventils 138 und des Pumpenventils 139 generiert.

Die Pumpe 140 ist als Saugpumpe konfiguriert, deren Saugseite 143 an das Ansaugvolumen 134 angeschlossen ist und deren Druckseite 144 mittelbar oder unmittelbar mit der Umgebung kommuniziert, um die Luft aus dem Ansaugvolumen 134 anzusaugen und in die Umgebung abzugeben. Die zu untersuchende Luft wird so entlang der durch den Pfeil 132 gekennzeichneten Richtung durch das Nachweismedium 130 hindurch gesaugt, wobei das Nachweisvolumen 128 sehr nah am Anfang des Strömungsverlaufs angeordnet ist, so dass die meisten passiven und aktiven Komponenten zur Erzeugung und Regulierung der Luftströmung 132, wie zum Beispiel die Pumpe 140, das Pumpenventil 138 sowie der Unterdrucktank 136, nicht mit den pathogenen Substanzen in Kontakt kommen und daher auch im positiven Testfall nicht dekontaminiert oder ausgetauscht werden müssen.

Weiterhin ist in dem Gehäuse 120 ein Sensorelement 146 angeordnet, das zum Detektieren einer Trübung und/oder Färbung in dem Nachweismedium 130 in Abhängigkeit von der Präsenz wenigstens einer pathogenen Substanz in der durch das Nachweismedium 130 hindurchgeströmten Luft eingerichtet ist. Das Sensorelement 146 ist ein mit der Steuerung 141 elektronisch verbundener optischen Sensor, der auf ein Sichtfenster 148 in dem Behälter ausgerichtet ist, welches durch einen lichtdurchlässigen Gehäusewandabschnitt gebildet sein kann.

Der Lufteinlass 124 wird durch eine in der hier dargestellten bestimmungsgemäßen Orientierung abschnittsweise, bezogen auf die Schwerkraftrichtung, nach unten in das Nachweisvolumen 128 eintauchende Zuleitung 150, auch als Tauchrohr bezeichnet, gebildet. Der Lufteinlass 124 des Behälters 122 mündet so unterhalb des Luftauslasses 126 in den Behälter 122, wodurch die Luft von oben zugeführt und unmittelbar in der Nachweisflüssigkeit 130 freigesetzt wird, von wo diese durch die Nachweisflüssigkeit 130 perlt und die enthaltenen Aerosole mit der Nachweisflüssigkeit 130 in Kontakt treten. Die Nachweisflüssigkeit 130 trennt die Lufteinlassmündung 152 fluidisch von der Luftauslassmündung 154. Das Puffervolumen 133 entspricht wenigstens dem Volumen der in dem eingetauchten Abschnitt des Tauchrohrs 150 beim Einsaugen anstehenden Luftsäule. Dadurch wird verhindert, dass die durch die Luft in dem Tauchrohrohr 150 verdrängte Nachweisflüssigkeit 130 den Füllspiegel soweit ansteigen lässt, dass die Nachweisflüssigkeit 130 in das Ansaugvolumen 134 gelangt.

Im Bereich des Lufteinlasses 124, genauer der Lufteinlassmündung 152, in dem Behälter 122 befindet sich eine poröse oder perforierte Luftverteilerstruktur 156, die der Verteilung der in das Nachweisvolumen 128 strömenden Luft auf möglichst kleine Gasblasen dient.

Stromaufwärts des Lufteinlasses 124 des Behälters 122 in der Einlassöffnung 129 angeordnet sind ein steuerbares Einlassventil 158 und weiter stromaufwärts ein weiterer Drucksensor 160. Der hiermit ermittelte Druck in der Einlassöffnung 129 wird mittels der Steuerung 141 mit programmierten Schaltschwellen verglichen, aus welchem Vergleich in Verbindung mit etwaigen weiteren Eingangsgrößen die Steuerung 141 ein Steuersignal zum Öffnen oder Schließen des Einlassventils 158 erzeugt.

Das Nachweisgerät 100 umfasst weiterhin ein mit der Einlassöffnung 129 verbundenes Mundstück 162. Das Mundstück 162 weist einen Einlassabschnitt 164, einen Auslassabschnitt 166 und einen abzweigenden Verbindungsabschnitt 168 auf. Der Verbindungsabschnitt 168 ist zur fluiddichten Verbindung mit der Einlassöffnung 129 eingerichtet. Einlassabschnitt 164, Auslassabschnitt 166 und Verbindungsabschnitt 168 sind dabei strömungstechnisch so gestaltet und dimensioniert, dass sich einerseits durch Ausatmen in den Einlassabschnitt 164 hinein ein ausreichender Überdruck in dem Verbindungsabschnitt 168 aufbauen lässt, um den programmierten Schwellenwert an dem Drucksensor 160 zu überschreiten. Andererseits muss gewährleistet sein, dass durch das Ausatmen auch kein wesentlich höherer Überdruck aufbaut, um den Volumenstrom durch das Nachweismedium 130 nicht erheblich zu manipulieren. Der meiste Anteil der ausgeatmeten Luft entweicht deshalb durch den Auslassabschnitt 166 in die Umgebung. Deshalb umfasst der Auslassabschnitt 166 ein die pathogene Substanz zurückhaltendes Filterelement 170, damit die das Nachweismedium 130 nicht passierende ausgeatmete Luft möglichst frei von etwaig vorhandenen pathogenen Substanzen in die Umgebung abgegeben wird.

Wird das Nachweisgerät 100 zur Analyse des Atems einer Person verwendet, dient die Kombination aus dem Einlassventil 158 und dem Drucksensor 160 dazu, zunächst festzustellen, dass eine Person in das Mundstück 162 atmet. Hierdurch erhöht sich der vor dem Einlassventil 158 anstehende Druck. Stellt der Sensor 160 fest, dass der voreingestellte Schwellenwert überschritten wird, öffnet die Steuerung 141 das Einlassventil 158, um das Mundstück 162 fluidisch mit dem Nachweisvolumen 128 zu verbinden. Der definierte Druckabfall zwischen dem so geregelten Überdruck im Mundstück 162 und dem wie oben beschrieben geregelten Unterdruck in dem Ansaugvolumen 134 sorgt für einen geregelten Volumenstrom des ausgeatmeten Aerosols.

In einer abgewandelten, vereinfachten Bauweise können der Drucksensor 160 und das Einlassventil 158 auch durch ein passives, in den Einlassabschnitt 164 des Mundstückes 162 integriertes Ventil ersetzt werden, das im Ruhezustand geschlossen ist und bei einem bestimmten Druckgefälle in Richtung des Nachweisvolumens öffnet. Das Ventil kann beispielsweise als Rückschlagventil mit eingestellter Vorspannung oder als Schlitzventil ausgebildet sein. Das Schlitzventil kann durch einen einfachen Schlauch, vorzugsweise aus Silikon, mit einem geschlossenen Ende gebildet werden. Nahe dem Ende befinden sich ein oder mehrere Einschnitte oder Schlitze in geeigneter Form, die sich bei einem bestimmten Druckgefälle von innen nach außen öffnen. Ist das Druckgefälle geringer oder verschwunden, bewirken die elastischen Rückstellkräfte des Schlauches, dass sich der oder die Schlitze verschließen. Ein Eindringen von Luft oder Flüssigkeit von außen in den Schlauch hinein wird so verhindert. Das Schlitzventil ist besonders einfach und kostengünstig.

Der Behälter 122 weist am Lufteinlass 124 und am Luftauslass 126 jeweils ein das Austreten des Nachweismediums 130 aus dem entnommenen Behälter 122 verhinderndes Verschlusselement 172 auf. Auch dieses Ventil kann als Gravitationsventil oder als Rückschlagventil mit eingestellter Vorspannung oder als Schlitzventil ausgebildet sein. Das Schlitzventil ist besonders einfach und kostengünstig. Es kann durch einen einfachen Schlauch, vorzugsweise aus Silikon, mit einem geschlossenen Ende gebildet werden. Nahe dem Ende befinden sich ein oder mehrere Einschnitte oder Schlitze in geeigneter Form, die sich bei einem bestimmten Druckgefälle von innen nach außen öffnen. Ist das Druckgefälle geringer oder verschwunden, bewirken die elastischen Rückstellkräfte des Schlauches, dass sich der oder die Schlitze verschließen. Ein Eindringen von Luft oder Flüssigkeit von außen in den Schlauch hinein wird so verhindert.

Die Pumpe 140, die Steuerung 141, die steuerbaren Ventile 138, 139 und 158, das Sensorelement 146, die Drucksensoren 142, 160, die Einlassöffnung 129, der Behälter 122 und der Unterdrucktank 136, sowie alle Verbindungsleitungen dazwischen sind in dem Gehäuse 120 beherbergt.

Nicht gezeigt ist eine ebenfalls in dem Gehäuse 120 angeordnete und mit der Steuerung 141 elektronisch verbundene Lichtquelle, beispielsweise in Form einer LED oder einer Laserdiode. Die Lichtquelle strahlt Licht durch ein in einer Wand des Behälters 122 im Bereich des Nachweisvolumens 128 angeordnetes Einlassfenster in das Nachweismedium 130 ein. Je nach Messverfahren sind die optischen Achsen des Sensorelements 146 und der Lichtquelle koaxial (Extinktions- oder Absorptionsmessung) oder rechtwinklig (Streulichtmessung) zueinander ausgerichtet.

Ferner kann in dem Gehäuse 120 eine Stromversorgung in Form einer Batterie, insbesondere eines aufladbaren Akkumulators, oder in Form einer nach außen geführte Leitung zum Anschluss an eine Stromquelle vorgesehen sein, beides nicht dargestellt. Weiterhin zwecks Vereinfachung nicht dargestellt können in oder an dem Gehäuse Anzeige- oder Datenübertragungsmodule angeordnet sein. Insbesondere kann das Nachweisgerät eine Schnittstelle und/oder ein Funkmodul für einen kabelgebundenen oder funkbasierten Daten- oder Signalaustausch mit einem Server oder einem stationären oder mobilen Endgerät oder mit gleichartigen Nachweisgeräten aufweisen. Alternativ oder zusätzlich kann das Nachweisgerät 100 auch mit einer optischen und/oder akustischen Anzeige ausgestattet sein.

Die Pumpe 140, die steuerbaren Ventile 138, 139 und 158, das Sensorelement 146, die Drucksensoren 142, 160, die Lichtquelle sind mit der Steuerung 141 elektronisch durch Steuerleitungen verbunden, welche zur Vereinfachung der Grafik nicht eingezeichnet sind.

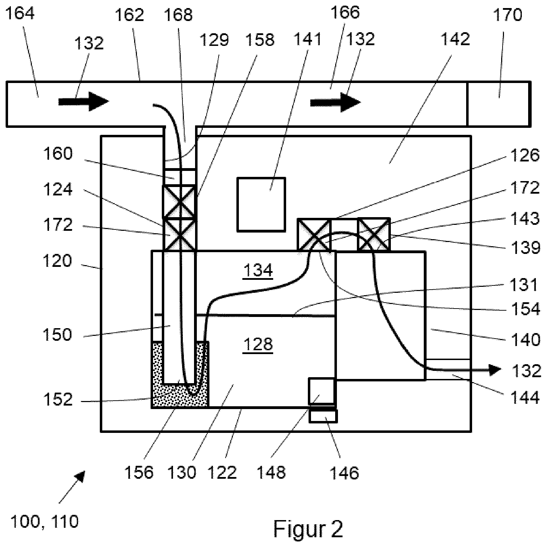

In Figur 2 ist eine andere Ausführungsform des Nachweisgeräts 100 für den Nachweis in Luft enthaltener pathogener Aerosolpartikel in der Verwendung als Testvorrichtung 110 für die ausgeatmete Luft eines Probanden vereinfacht dargestellt. Das Nachweisgerät 100 unterscheidet sich von der Ausführungsform der Figur 1 im Prinzip dadurch, dass das Ansaugvolumen 134 in dem Behälter 122 integriert ist. Dadurch entfallen der Unterdrucktank 136, die Anschlussleitung 137 und das Ansaugventil 138. Das Nachweisvolumen 128 und das Ansaugvolumen 134 bilden hierdurch gemeinsam das Behältervolumen, wobei das Ansaugvolumen 134 in der dargestellten bestimmungsgemäßen Orientierung oberhalb des Nachweisvolumens 128, d. h. über dem Füllspiegel 131 der Nachweisflüssigkeit 130, in dem Behältervolumen gebildet wird. Der Luftauslass 126 mündet daher in das Ansaugvolumen 134 bzw. geht von diesem ab. Das Ansaugvolumen 134 ist in der Regel wesentlich größer als das Puffervolumen 133 gemäß dem Beispiel aus Figur 1, weil das Ansaugvolumen 134 als Unterdruckdruckspeicher auch über eine längere Ansaugdauer eine möglichst konstante Druckdifferenz bereitstellen sollte.

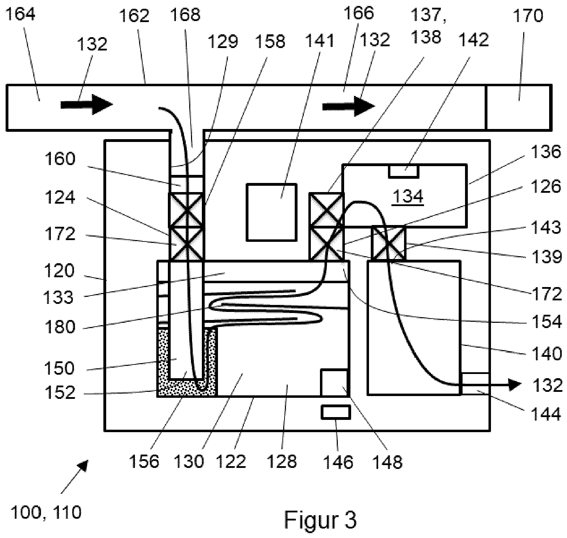

In Figur 3 ist noch eine andere Ausführungsform des Nachweisgeräts 100 für den Nachweis in Luft enthaltener pathogener Aerosolpartikel in der Verwendung als Testvorrichtung 110 für die ausgeatmete Luft eines Probanden vereinfacht dargestellt. Das Nachweisgerät unterscheidet sich von der Ausführungsform der Figur 1 dadurch, dass in dem Behälter 120 innerhalb des Nachweisvolumens 128 drei Luftleitelemente 180 der Gestalt angeordnet sind, dass sie eine mäanderförmige Struktur bilden. Der Luftstrom 132 durch das Nachweismedium 130 vom Lufteinlass 124 zum Luftauslass 126 wird dadurch mehrfach umgelenkt. Das Nachweismedium 130 wird also nicht auf direktem Weg vom Lufteinlass 124 zum Luftauslass 126 durchströmt, sondern auf einem verlängerten Weg, wodurch sich die Kontaktzeit zwischen der Luft bzw. den darin enthaltene Aerosolen und im Ergebnis auch den darin enthaltenen Viren und dem Nachweismedium 130 verlängert und dadurch die Nachweiseffizienz des Nachweisgerätes 100 erhöht.

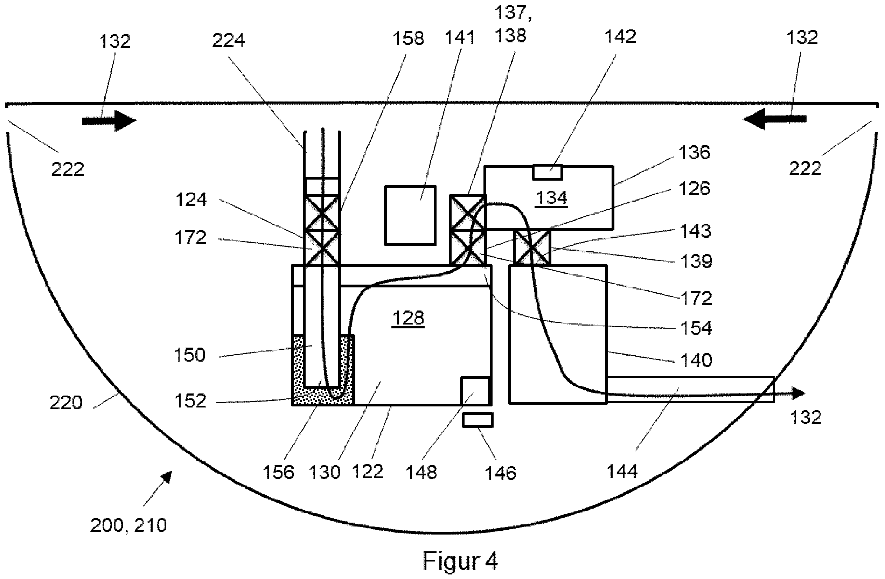

In Figur 4 ist eine Ausführungsform des Nachweisgeräts 200 für den Nachweis in Luft enthaltener pathogener Aerosolpartikel in der Verwendung als Überwachungsvorrichtung 210 für Raumluft vereinfacht dargestellt. Das Nachweisgerät 200 unterscheidet sich von der Ausführungsform der Figur 1 im Wesentlichen durch die Form des Gehäuses 220, welches hier beispielhaft als kugelsegmentförmige Schale zur Deckenmontage ausgebildet ist. Ferner fehlt in dieser Ausgestaltung das Mundstück 162. Die Luft wird durch Öffnungen 222 in der Gehäusewandung in das Gehäuseinnere und dann in ein Ansaugrohr 224 eingesaugt, welches in den Lufteinlass 124 mündet. Der Drucksensor 160 kann hier entfallen, weil bei dieser Verwendung im Ansaugrohr zunächst stets Umgebungsdruck ansteht. Der Ansaugvorgang wird durch die Steuerung initiiert, indem die Pumpe aktiviert wird und bei Erreichen eines voreingestellten Unterdruckes in Ansaugvolumen 134 bzw. im Unterdrucktank 136 das Einlassventil 158 öffnet. Die folgende Messung läuft analog wie zuvor im Zusammenhang mit den Testvorrichtungen 110 beschrieben. Die Messungen können in der Verwendung Nachweisgeräts 200 als Überwachungsvorrichtung 210 nach einem festgelegten Schema in bestimmten Zeitabständen erfolgen.

### BEZUGSZEICHENLISTE

- 100: Nachweisgerät
- 110: Testvorrichtung
- 120: Gehäuse
- 122: Behälter
- 124: Lufteinlass
- 126: Luftauslass
- 128: Nachweisvolumen
- 129: Einlassöffnung
- 130: Nachweismedium, Nachweisflüssigkeit
- 131: Füllspiegel
- 132: Pfeil, Luftströmung
- 133: Puffervolumen
- 134: Ansaugvolumen
- 136: Unterdrucktank
- 137: Anschlussleitung
- 138: Ansaugventil
- 139: Pumpenventil
- 140: Pumpe
- 141: Steuerung
- 142: Drucksensor
- 143: Saugseite der Pumpe
- 144: Druckseite der Pumpe
- 146: Sensorelement
- 148: Sichtfenster
- 150: Zuleitung, Tauchrohr
- 152: Lufteinlassmündung
- 154: Luftauslassmündung
- 156: Luftverteilerstruktur
- 158: Einlassventil
- 160: Drucksensor
- 162: Mundstück
- 164: Einlassabschnitt
- 166: Auslassabschnitt
- 168: Verbindungsabschnitt
- 170: Filterelement
- 172: Verschlusselement
- 180: Luftleitelement

- 200: Nachweisgerät
- 210: Überwachungsvorrichtung
- 220: Gehäuse
- 222: Öffnung
- 224: Ansaugrohr

## Patentansprüche

1. Behälter (122) mit einem Lufteinlass (124), einem Luftauslass (126), einem Nachweisvolumen (128) und einer Nachweisflüssigkeit (130) für den Nachweis in Luft enthaltener pathogener Substanzen, insbesondere Viren, wobei die Nachweisflüssigkeit (130) bei bestimmungsgemäßem Gebrauch so in dem Nachweisvolumen (128) platziert ist, dass eine Luftströmung (132) innerhalb des Behälters (122) vom Lufteinlass (124) zum Luftauslass (126) die Nachweisflüssigkeit (130) durchströmt und wobei der Lufteinlass (124), bezogen auf die Schwerkraftrichtung, unterhalb des Luftauslasses (126) in den Behälter (122) mündet.

2. Behälter (122) nach Anspruch 1, **dadurch gekennzeichnet,**
**dass** die Nachweisflüssigkeit (130) Antikörper oder mit Antikörpern beladene Latexpartikel enthält.

3. Nachweisgerät (100, 200) für den Nachweis in Luft enthaltener pathogener Substanzen, insbesondere Viren, aufweisend:
einen Behälter (122) nach einem der Ansprüche 1 oder 2,
ein Ansaugvolumen (134) stromabwärts des Nachweisvolumens (128),
eine mit dem Ansaugvolumen (134) verbundene Pumpe (140) zum Erzeugen eines Unterdruckes in dem Ansaugvolumen (134),
und ein Sensorelement (146), eingerichtet zum Detektieren einer Trübung und/oder Färbung in der Nachweisflüssigkeit (130) in Abhängigkeit von der Präsenz wenigstens einer pathogenen Substanz in der durch die Nachweisflüssigkeit (130) hindurchgeströmten Luft.

4. Nachweisgerät (100, 200) nach Anspruch 3, **dadurch gekennzeichnet,**
**dass** der Lufteinlass (124) durch eine bei bestimmungsgemäßem Gebrauch abschnittsweise, bezogen auf die Schwerkraftrichtung, nach unten in das Nachweisvolumen (128) eintauchende Zuleitung (150) gebildet wird.

5. Nachweisgerät (100, 200) nach Anspruch 3 oder 4, **dadurch gekennzeichnet,**
**dass** sich im Bereich des Lufteinlasses (124) eine poröse oder perforierte Luftverteilerstruktur (156) befindet, die durch eine granulare Substanz oder ein offenporiges Material gebildet wird.

6. Nachweisgerät (100, 200) nach einem der Ansprüche 3 bis 5, **dadurch gekennzeichnet,**
**dass** das Ansaugvolumen (134) durch einen Unterdrucktank (136) gebildet wird, der über eine Anschlussleitung (137) mit dem Luftauslass (126) des Behälters (122) verbunden ist.

7. Nachweisgerät (100, 200) nach einem der Ansprüche 3 bis 6, **dadurch gekennzeichnet,**
**dass** das Ansaugvolumen (134) in dem Behälter (122) integriert ist, wobei das Nachweisvolumen (128) und das Ansaugvolumen (134) gemeinsam das Behältervolumen bilden, wobei das Ansaugvolumen (134) bei bestimmungsgemäßem Gebrauch, bezogen auf die Schwerkraftrichtung, oberhalb des Nachweisvolumens (128) in dem Behältervolumen gebildet wird und wobei der Luftauslass (126) in das Ansaugvolumen (134) mündet.

8. Nachweisgerät (100, 200) nach einem der Ansprüche 3 bis 7, **gekennzeichnet durch,**
eine Steuerung (141) und einen Drucksensor (142), der in dem Ansaugvolumen (134) angeordnet oder mit diesem unmittelbar fluidisch verbunden ist, wobei die Pumpe (140) und der Drucksensor (142) mit der Steuerung (141) elektronisch verbunden sind.

9. Nachweisgerät (100, 200) nach einem der Ansprüche 3 bis 8, **gekennzeichnet durch,**
eine Steuerung (141) und ein steuerbares Pumpenventil (139), das zwischen der Pumpe (140) und dem Ansaugvolumen (134) angeordnet ist, wobei die Pumpe (140) und das steuerbares Pumpenventil (139) mit der Steuerung (141) elektronisch verbunden sind.

10. Nachweisgerät (100, 200) nach einem der Ansprüche 3 bis 9, **gekennzeichnet durch,**
eine Steuerung (141), ein Gehäuse (120), in dem der Behälter (122) angeordnet ist und das einen Einlassstutzen oder eine Einlassöffnung (129) aufweist, der oder die fluidisch mit dem Lufteinlass (124) des Behälters (122) kommuniziert, und ein steuerbares Einlassventil (158), das in dem Einlassstutzen angeordnet ist, wobei die Pumpe (140) und das steuerbare Einlassventil (158) mit der Steuerung (141) elektronisch verbunden sind.

11. Nachweisgerät (100, 200) nach Anspruch 10, **gekennzeichnet durch,**
einen Drucksensor (160), der stromaufwärts des Einlassventils (158) in dem Einlassstutzen angeordnet oder mit diesem unmittelbar fluidisch verbunden ist und der mit der Steuerung (141) elektronisch verbunden ist.

12. Nachweisgerät (100, 200) nach einem der Ansprüche 10 oder 11, **dadurch gekennzeichnet,**
**dass** der Behälter (122) und das Gehäuse (120) korrespondierende Verriegelungselemente aufweisen, wobei der Behälter (122) aus dem Gehäuse (120) entnehmbar ist, wenn die Verriegelungselemente nicht in Eingriff stehen, und in dem Gehäuse (120) in seiner bestimmungsgemäßen Position arretiert ist, wenn die Verriegelungselemente in Eingriff stehen, wobei der Behälter (122) am Lufteinlass (124) und am Luftauslass (126) jeweils ein das Austreten der Nachweisflüssigkeit (130) aus dem entnommenen Behälter (122) verhinderndes Verschlusselement (172) aufweist.

13. Nachweisgerät (100, 200) nach einem der Ansprüche 10 bis 12, **gekennzeichnet durch,**
ein mit dem Einlassstutzen verbindbares Mundstück (162), das einen Einlassabschnitt (164), einen Auslassabschnitt (166) und einen abzweigenden Verbindungsabschnitt (168) umfasst, wobei der Verbindungsabschnitt (168) zur fluidischen Verbindung mit den Einlassstutzen eingerichtet ist und wobei der Auslassabschnitt (166) ein die pathogene Substanz zurückhaltendes Filterelement (170) umfasst.

14. Nachweisgerät (100, 200) nach einem der Ansprüche 3 bis 13, **dadurch gekennzeichnet,**
**dass** das Sensorelement (146) ein mit der Steuerung (141) elektronisch verbundener optischen Sensor ist und dass in einer Wand des Behälters (122) im Bereich des Nachweisvolumens (128) ein Sichtfenster (148) angeordnet ist, auf das der optische Sensor ausgerichtet ist und
**dass** eine mit der Steuerung (141) elektronisch verbundene Lichtquelle und ein in einer Wand des Behälters (122) im Bereich des Nachweisvolumens (128) angeordnetes Einlassfenster vorgesehen sind, auf das die Lichtquelle ausgerichtet ist, wobei die Lichtquelle, der optische Sensor und die Steuerung (141) zur Bestimmung des in der Nachweisflüssigkeit (130) absorbierten oder gestreuten Lichtes eingerichtet sind.

15. Verfahren zum Nachweis in Luft enthaltener pathogener Substanzen (Aerosolpartikel), insbesondere Viren, aufweisend
- Bereitstellen eines Behälters (122) nach einem der Ansprüche 1 oder 2 mit einem Lufteinlass (124), einem Luftauslass (126) und einem Nachweisvolumen (128),
- Bereitstellen einer Nachweisflüssigkeit (130) in dem Nachweisvolumen (128),
- Erzeugen eines Unterdruckes (bezogen auf den Umgebungsdruck) in einem Ansaugvolumen (134) stromabwärts des Nachweisvolumens (128) mittels einer Pumpe (140),
- Ansaugen von Luft vom Lufteinlass (124) durch die Nachweisflüssigkeit (130) hindurch,
- Detektieren einer Trübung und/oder Färbung in dem Nachweismedium (130) mittels eines Sensorelements (146) in Abhängigkeit von der Präsenz wenigstens einer pathogenen Substanz in der hindurchgesaugten Luft.

## Claims

1. Container (122) with an air inlet (124), an air outlet (126), a detection volume (128) and a detection liquid (130) for the detection of pathogenic substances contained in air, in particular viruses, wherein the detection liquid (130) is placed in the detection volume (128) when used as intended, such that an air flow (132) within the container (122) flows through the detection liquid (130) from the air inlet (124) to the air outlet (126) and wherein the air inlet (124) opens into the container (122) below the air outlet (126), with respect to the direction of gravity.

2. Container (122) according to claim 1, **characterized in that**
the detection liquid (130) contains antibodies or antibody-loaded latex particles.

3. Detection device (100, 200) for the detection of pathogenic substances contained in air, in particular viruses, comprising
a container (122) according to one of claims 1 or 2,
an intake volume (134) downstream of the detection volume (128),
a pump (140) connected to the intake volume (134) for generating a vacuum in the intake volume (134),
and a sensor element (146) arranged to detect turbidity and/or coloration in the detection liquid (130) as a result of the presence of at least one pathogenic substance in the air flowing through the detection liquid (130).

4. Detection device (100, 200) according to claim 3, **characterized in that,**
the air inlet (124) is formed by a supply line (150) which, when used as intended, partly dips downwards into the detection volume (128) with respect to the direction of gravity.

5. Detection device (100, 200) according to claim 3 or 4, **characterized in that,**
a porous or perforated air distributor structure (156) is located in the region of the air inlet (124), which is formed by a granular substance or an open-pored material.

6. Detection device (100, 200) according to one of claims 3 to 5, **characterized in that**
the intake volume (134) is formed by a vacuum tank (136), which is connected to the air outlet (126) of the container (122) via a connection pipe (137).

7. Detection device (100, 200) according to one of claims 3 to 6, **characterized in that**
the intake volume (134) is integrated in the container (122), wherein the detection volume (128) and the intake volume (134) together form the container volume, wherein, when used as intended, the intake volume (134) is formed above the detection volume (128) in the container volume, with respect to the direction of gravity, and wherein the air outlet (126) opens into the intake volume (134).

8. A detection device (100, 200) according to any one of claims 3 to 7, **characterized by,**
a controller (141) and a pressure sensor (142), which is arranged in the intake volume (134) or is directly fluidically connected thereto, the pump (140) and the pressure sensor (142) being electronically connected to the controller (141).

9. A detection device (100, 200) according to any one of claims 3 to 8, **characterized by,**
a controller (141) and a controllable pump valve (139) arranged between the pump (140) and the intake volume (134), the pump (140) and the controllable pump valve (139) being electronically connected to the controller (141).

10. A detection device (100, 200) according to any one of claims 3 to 9, **characterized by,**
a controller (141), a housing (120) in which the container (122) is disposed and which has an inlet port or opening (129) in fluid communication with the air inlet (124) of the container (122), and a controllable inlet valve (158) disposed in the inlet port, wherein the pump (140) and the controllable inlet valve (158) are electronically connected to the controller (141).

11. Detection device (100, 200) according to claim 10, **characterized by,**
a pressure sensor (160), which is arranged in the inlet connection piece or is directly fluidically connected thereto upstream of the inlet valve (158) and which is electronically connected to the control unit (141).

12. Detection device (100, 200) according to one of claims 10 or 11, **characterized in that,**
the container (122) and the housing (120) have corresponding locking elements, wherein the container (122) is removable from the housing (120) when the locking elements are not engaged and wherein it is locked in its intended position in the housing (120) when the locking elements are engaged, wherein the container (122) has a closure element (172) at the air inlet (124) and at the air outlet (126) which prevents the detection liquid (130) from flowing out of the removed container (122).

13. A detection device (100, 200) according to any one of claims 10 to 12, **characterized by,**
a mouthpiece (162) connectable to the inlet port and comprising an inlet section (164), an outlet section (166) and a branching connection section (168), wherein the connection section (168) is adapted for fluidic connection to the inlet port and wherein the outlet section (166) comprises a filter element (170) retaining the pathogenic substance.

14. Detection device (100, 200) according to any one of claims 3 to 13, **characterized in that,**
the sensor element (146) is an optical sensor which is electronically connected to the controller (141), and **in that** a viewing window (148), towards which the optical sensor is aligned, is arranged in a wall of the container (122) in the region of the detection volume (128), and
**in that** a light source electronically connected to the controller (141) and an inlet window arranged in a wall of the container (122) in the region of the detection volume (128) are provided, towards which the light source is aligned, wherein the light source, the optical sensor and the controller (141) being set up to determine the light absorbed or scattered in the detection liquid (130).

15. Method for the detection of pathogenic substances (aerosol particles) contained in air, in particular viruses, comprising
- providing a container (122) according to any one of claims 1 or 2 with an air inlet (124), an air outlet (126) and a detection volume (128),
- providing a detection liquid (130) in the detection volume (128),
- generating a negative pressure (relative to the ambient pressure) in an intake volume (134) downstream of the detection volume (128) by means of a pump (140),
- intaking air from the air inlet (124) through the detection liquid (130),
- detecting turbidity and/or coloration in the detection medium (130) by means of a sensor element (146) as a result of the presence of at least one pathogenic substance in the air sucked through.

## Revendications

1. Contenant (122) comprenant une admission d'air (124), une sortie d'air (126), un volume de détection (128) et un liquide de détection (130) pour la détection de substances pathogènes contenues dans l'air, en particulier de virus, dans lequel le liquide de détection (130), lors d'une utilisation prévue, est placé dans le volume de détection (128) de telle sorte qu'un flux d'air (132) à l'intérieur du contenant (122) traverse le liquide de détection (130) de l'admission d'air (124) à la sortie d'air (126), et dans lequel l'admission d'air (124), en considérant la direction de la gravité, débouche dans le contenant (122) en dessous de la sortie d'air (126).

2. Contenant (122) selon la revendication 1, **caractérisé en ce que** le liquide de détection (130) contient des anticorps ou des particules de latex chargées d'anticorps.

3. Appareil de détection (100, 200) pour la détection de substances pathogènes contenues dans l'air, en particulier de virus, présentant :
un contenant (122) selon l'une des revendications 1 ou 2,
un volume d'aspiration (134) en aval du volume de détection (128), une pompe (140) reliée au volume d'aspiration (134) et permettant de générer une dépression dans le volume d'aspiration (134),
et un élément capteur (146), conçu pour détecter une turbidité et/ou une coloration dans le liquide de détection (130) en fonction de la présence d'au moins une substance pathogène dans l'air traversant le liquide de détection (130).

4. Appareil de détection (100, 200) selon la revendication 3,
**caractérisé en ce que**
l'admission d'air (124) est formée par une conduite d'alimentation (150) qui, en considérant la direction de la gravité, plonge vers le bas dans le volume de détection (128), au moins par segments, dans le cas d'une utilisation conforme.

5. Appareil de détection (100, 200) selon la revendication 3 ou 4,
**caractérisé ce que**
dans la zone de l'admission d'air (124) se trouve une structure de distribution d'air poreuse ou perforée (156) qui est formée par une substance granulaire ou un matériau à pores ouverts.

6. Appareil de détection (100, 200) selon l'une des revendications 3 à 5, **caractérisé en ce que**
le volume d'aspiration (134) est formé par un réservoir à dépression (136) qui est relié à la sortie d'air (126) du contenant (122) par une conduite de raccordement (137).

7. Appareil de détection (100, 200) selon l'une des revendications 3 à 6, **caractérisé en ce que**
le volume d'aspiration (134) est intégré dans le contenant (122), le volume de détection (128) et le volume d'aspiration (134) formant ensemble le volume de contenant, le volume d'aspiration (134) étant formé dans le volume de contenant, dans le cas d'une utilisation conforme, en considérant la direction de la gravité, au-dessus du volume de détection (128), et la sortie d'air (126) débouchant dans le volume d'aspiration (134).

8. Appareil de détection (100, 200) selon l'une des revendications 3 à 7, **caractérisé par**
une commande (141) et un capteur de pression (142) qui est agencé dans le volume d'aspiration (134) ou qui y est directement relié par un fluide, la pompe (140) et le capteur de pression (142) étant reliés électroniquement à la commande (141).

9. Appareil de détection (100, 200) selon l'une des revendications 3 à 8, **caractérisé par**
une commande (141) et une vanne de pompe pouvant être commandée (139) agencée entre la pompe (140) et le volume d'aspiration (134), la pompe (140) et la vanne de pompe pouvant être commandée (139) étant reliées électroniquement à la commande (141).

10. Appareil de détection (100, 200) selon l'une des revendications 3 à 9, **caractérisé par**
une commande (141), un boîtier (120) dans lequel est agencé le contenant (122) et qui présente une tubulure d'entrée ou un orifice d'entrée (129) qui communique de manière fluidique avec l'admission d'air (124) du contenant (122), et une vanne d'entrée pouvant être commandée (158) agencée dans la tubulure d'entrée, la pompe (140) et la vanne d'entrée pouvant être commandée (158) étant reliées électroniquement à la commande (141).

11. Appareil de détection (100, 200) selon la revendication 10,
**caractérisé par**
un capteur de pression (160) qui est agencé en amont de la vanne d'entrée (158) dans la tubulure d'entrée ou directement relié à celle-ci de manière fluidique, et qui est relié électroniquement à la commande (141).

12. Appareil de détection (100, 200) selon l'une des revendications 10 à 11, **caractérisé en ce que**
le contenant (122) et le boîtier (120) présentent des éléments de verrouillage correspondants, le contenant (122) pouvant être retiré du boîtier (120) lorsque les éléments de verrouillage ne sont pas en prise et étant bloqué dans le boîtier (120) dans sa position prévue lorsque les éléments de verrouillage sont en prise, le contenant (122) présentant, au niveau de l'admission d'air (124) et au niveau de la sortie d'air (126), respectivement, un élément de fermeture (172) empêchant la sortie du liquide de détection (130) hors du contenant (122) retiré.

13. Appareil de détection (100, 200) selon l'une des revendications 10 à 12, **caractérisé par**
un embout buccal (162) pouvant être relié à la tubulure d'entrée, qui présente une section d'entrée (164), une section de sortie (166) et une section de connexion de dérivation (168), la section de connexion (168) étant conçue pour une connexion fluidique avec les tubulures d'entrée, et la section de sortie (166) comprenant un élément filtrant (170) qui retient la substance pathogène.

14. Appareil de détection (100, 200) selon l'une des revendications 3 à 13, **caractérisé en ce que**
l'élément capteur (146) est un capteur optique relié électroniquement à la commande (141), et **en ce qu'**une fenêtre de visualisation (148) avec laquelle le capteur optique est aligné, est agencée dans une paroi du contenant (122), dans la zone du volume de détection (128), et
**en ce que** sont prévues une source de lumière reliée électroniquement à la commande (141) et une fenêtre d'entrée agencée dans une paroi du contenant (122), dans la zone du volume de détection (128), vers laquelle la source de lumière est orientée, la source de lumière, le capteur optique et la commande (141) étant agencés pour déterminer la lumière absorbée ou diffusée dans le liquide de détection (130).

15. Procédé de détection de substances pathogènes contenues dans l'air (particules d'aérosol), en particulier de virus, comprenant les étapes consistant à
- mettre à disposition un contenant (122) selon l'une des revendications 1 ou 2 présentant une admission d'air (124), une sortie d'air (126) et un volume de détection (128),
- mettre à disposition un liquide de détection (130) dans le volume de détection (128),
- générer une dépression (par rapport à la pression ambiante) dans un volume d'aspiration (134) en aval du volume de détection (128) au moyen d'une pompe (140),
- aspirer de l'air depuis l'admission d'air (124) à travers le liquide de détection (130),
- détecter une turbidité et/ou une coloration dans le milieu de détection (130) à l'aide d'un élément capteur (146) en fonction de la présence d'au moins une substance pathogène dans l'air aspiré.
